# EUROPEAN PATENT SPECIFICATION

(11) **EP 3 725 355 B1**
(45) Date of publication and mention of the grant of the patent: **04.05.2022**
(21) Application number: 20164018.2
(22) Date of filing: 18.03.2020
(51) Int. Cl.: A61M 25/00, A61M 27/00

(54) **PACKAGED PRECISION-LUBRICATED READY-TO-USE INTERMITTENT URINARY CATHETER**
VERPACKTER, PRÄZISIONSGESCHMIERTER, GEBRAUCHSFERTIGER INTERMITTIERENDER HARNKATHETER
CATHÉTER URINAIRE INTERMITTENT PRÊT À L'EMPLOI, LUBRIFIÉ AVEC PRÉCISION ET EMBALLÉ

(30) Priority: 16.04.2019 US 201916385440
(43) Date of publication of application: 21.10.2020
(73) Proprietor: Cure Medical, LLC, Newport Beach, CA 92663 (US)
(72) Inventor: Palmer, Timothy, Stillwater, MN 55082 (US)
(74) Representative: Vossius & Partner Patentanwälte Rechtsanwälte mbB

(56) References cited:
- EP-A1- 2 468 346
- EP-A1- 2 609 956
- GB-A- 2 561 843
- US-A- 6 059 107
- US-B1- 6 739 112

## Description

### BACKGROUND

Intermittent catheters are typically used by patients suffering from urinary incontinence or by individuals unable to enjoy voluntary urination. In our highly mobile culture, the ability to have the freedom to leave home for the day or longer is an important part of life. To accommodate this need single use, pre-lubricated catheters have been developed to allow patients to perform self catheterization. An individual requiring catheterization will typically utilize several catheters each and every day. This results in the usage of a large number of catheters over time, driving a demand for inexpensive catheters without sacrificing safety and comfort.

The high daily utilization rate for intermittent urinary catheters also results in the need for individuals requiring catheterization to transport several catheters with them whenever they leave the house for any extended period of time. Packaged catheters tend to be large and bulky, rendering it difficult to discretely transport a supply of catheters.

Accordingly, a need exists for an inexpensive and efficiently packaged intermittent urinary catheter, particularly one that is self-contained and ready for use immediately upon removal from the packaging.

A particular complication encountered with the production and transport of inexpensive, single use, pre-lubricated, ready for use catheters for self-catheterization is constraining the lubricant to the insertion portion of the packaged catheter so as to prevent lubrication of the fixture end portion of the catheter which must be gripped during insertion and removal of the catheter.

Accordingly, a particular need exists for an inexpensive method for packaging a single use, pre-lubricated, ready for use catheter for self-catheterization that constrains the lubricant to contact with the insertion portion of the packaged catheter during packaging, and results in a packaged catheter that restrains migration of the lubricant within the package into contact with the fixture end of the catheter under normal storage and transport conditions.

EP 2 609 956 A1 relates to a catheter assembly, comprising a catheter and an elongate package accommodating said catheter in a closed condition. The package is provided with an insert opening arranged to releasably receive an end of the elongate package in the closed condition, thereby temporarily retaining said closed package in a folded disposition in which also the catheter is folded.

EP 2 468 346 A1 relates to a catheter assembly, comprising a catheter, such as a urinary catheter, which at least a partly is provided with a hydrophilic coating. The catheter assembly further comprises a wetting fluid. The receptacle is in direct contact with the hydrophilic coating of the catheter over essentially the entire length of the part of the catheter being provided with the hydrophilic coating. This may e.g. be achieved by using a shrink wrap material. In order to accomplish adequate wetting, channels or the like may be provided on the inner surface of the receptacle or the outer surface of the catheter.

GB 2 561 843 A relates to a catheter assembly comprising a main body having a first opening, a second opening and a cavity defined therebetween configured to receive a lubricant. A catheter tube extends through the first opening, the cavity and the second opening. A seal configured to fluidly seal the second opening when the catheter tube is extended through the second opening. The main body comprises one or more alignment portions configured to radially constrain the catheter tube when it is received by the cavity. The alignment portions extend radially inwardly from an internal surface of the main body. The assembly may also comprise a catheter grip which may fluidly seal the first opening. The alignment portions may be discontinuous along the axial length of the cavity. The lubricant may comprise a gel. A container may hermetically seal at least a portion of the catheter tube. The container may be flexible and may comprise a foil. The seal may comprise a flexible or elastomeric material.

US 6,739,112 B1 relates to a system for packaging a bone allograft for use in a future medical procedure in which the bone allograft is stored in a saturated saline solution in an airtight container. The saline solution keeps the allograft hydrated and may be saturated with a combination of calcium, phosphate, or magnesium to inhibit mineral leaching out of the allograft during storage. The container may be deformable to conform to the shape of the allograft material as the container is "shrink-fitted" to the allograft. Optionally, the bone allograft may also be freeze dried prior to placement in the container.

US 6,059,107 relates to a urinary catheter assembly comprising a urinary catheter having on at least a part of its surface a hydrophilic surface layer intended to produce a low-friction surface character of the catheter by treatment with a liquid swelling medium prior to use of the catheter and a catheter package having a cavity for accommodation of the catheter. The package is made with walls of a gas impermeable material to accommodate a catheter pretreated with said liquid swelling medium for long time preservation of said low-friction surface character and provision of a ready-to-use catheter assembly.

### SUMMARY OF THE INVENTION

The invention for which protection is sought is defined by the independent claim. The dependent claims concern particular embodiments.

A first aspect of the invention is a method for efficiently packaging a ready to use intermittent urinary catheter. The packaged catheter includes, and except for appropriate labeling and marking preferably only includes, (i) an intermittent urinary catheter, (ii) packaging formed from first and second layers of film, and (iii) a lubricant. The catheter has a longitudinal axial length, an insertion end, a funnel end and a top view profile, and is hermetically packaged between the first and second layers of film within a retention chamber formed from the films. The retention chamber has a top view profile conforming to the top view profile of the catheter and retains a supply of lubricant. In an alternative embodiment, the lubricant is predominately retained within an enlarged lubricant retaining compartment formed in the retention chamber intermediate the insertion and funnel ends of the catheter.

A second aspect of the invention is a method of efficiently packaging a precision lubricated ready to use intermittent urinary catheter having an insertion end, an insertion end length, a fixture end, and a fixture end length. The method includes the steps of (a) obtaining a base film having a pocket with a first end and a second end, wherein the pocket is configured to retain the intermittent urinary catheter with the insertion end proximate the first end and the fixture end proximate the second end, (b) injecting a limited amount of lubricant into the pocket proximate the first end of the pocket so as to form a lubricant containing pocket, (c) placing the intermittent urinary catheter into the lubricant containing pocket so as to form a catheter containing pocket, with the insertion end of the intermittent urinary catheter proximate the first end and the fixture end of the intermittent urinary catheter proximate the second end, (d) sealing a cover film to the base film with an unsealed opening proximate the second end of the pocket so as to form an enclosed catheter containing retention chamber accessible through the opening, (e) drawing a vacuum on the enclosed catheter containing retention chamber through the opening sufficient to compress the retention chamber and cause lubricant to circumferentially flow around and coat a portion of the exterior surface area of the insertion end length of the catheter within the retention chamber without reaching and coating the fixture end length of the catheter within the retention chamber, and thereafter (f) sealing the opening so as to form a hermetically packaged intermittent urinary catheter having a circumferentially lubricated insertion end length and a lubricant-free fixture end length.

### BRIEF DESCRIPTION OF THE DRAWINGS

Figure 1 is a top view of one embodiment of the invention.
Figure 2 is a top view of another embodiment of the invention
Figure 3 is a side view of the invention depicted in Figure 2.

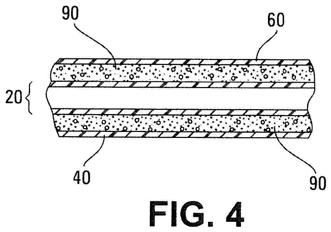

Figure 4 is an enlarged cross-sectional side view of a portion of the invention depicted in Figures 2 and 3 taken along line 4-4.
Figure 5 is a perspective view of one embodiment of a base layer and a cover layer suitable for use in hermetically packaging an intermittent urinary catheter in accordance with the invention.
Figure 6 is a perspective view of the base layer depicted in Figure 5 after injection of lubricant into the pocket of the base layer in accordance with the invention.
Figure 7 is a side view of the base layer depicted in Figure 6 after placement of a catheter into the lubricant containing pocket of the base layer and heat sealing of the cover layer to the base layer, but prior to pulling of a vacuum in accordance with the invention.
Figure 8 is a side view of the invention depicted in Figure 7 after pulling of a vacuum and complete sealing of the catheter within the packaging in accordance with the invention.

### DETAILED DESCRIPTION OF A PREFERRED EMBODIMENT

### Nomenclature

- **10**: Packaged Intermittent Urinary Catheter
- **20**: Catheter
- **20_{Pf}**: Top View Profile of Catheter
- **20x**: Axial Length of Catheter
- **21**: Insertion End of Catheter
- **21x**: Axial Length of Insertion End Portion of Catheter
- **22**: Fixture End or Funnel End of Catheter
- **22x**: Axial Length of Fixture End Portion of Catheter
- **25x**: Pre-vacuum Axial Length of Catheter In Contact with Lubricant Within the Pocket (Alpha Length)
- **30**: Fixture or Funnel
- **40**: Base Layer of Packaging
- **50**: Pocket in Base Layer of Packaging
- **50_{Pf}**: Top View Profile of Pocket
- **51**: First End of Pocket
- **52**: Second End of Pocket
- **60**: Cover Layer of Packaging
- **70**: Packaging
- **70_{Py}**: Peripheral Edge of Packaging
- **75**: Margins of Packaging
- **75w**: Width of Margins
- **80**: Retention Chamber
- **80_{Pf}**: Top View Profile of Retention Chamber
- **80_{Py}**: Periphery of Retention Chamber
- **80_{Lube}**: Lubricant Containing Compartment
- **80_{Neck}**: Metering Neck Area of Retention Chamber
- **81**: First End of Retention Chamber
- **82**: Second End of Retention Chamber
- **83**: Sides of Retention Chamber
- **90**: Lubricant
- x: Longitudinal or Axial Direction
- **y**: Lateral (Radial) Direction
- **z**: Transverse (Radial) Direction

### Definitions

As utilized herein, including the claims, the term *"**fixture**"* means and refers to the well known commercially available components commonly attached to the proximal non-insertion end of urinary catheters, including specifically but not exclusively funnels, luer locks, clamps, valves, etc.

As utilized herein, including the claims, the term *"**predominant**"* means at least 80%.

### Description

### PACKAGED CATHETER

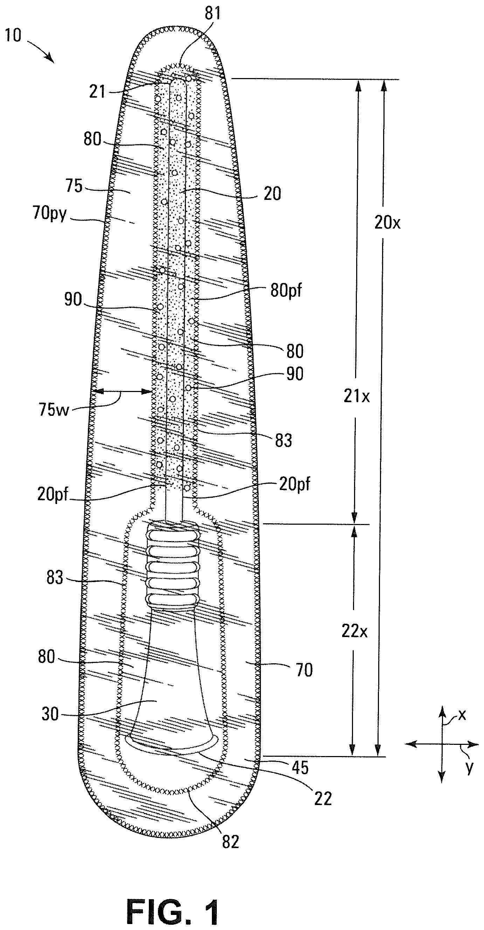

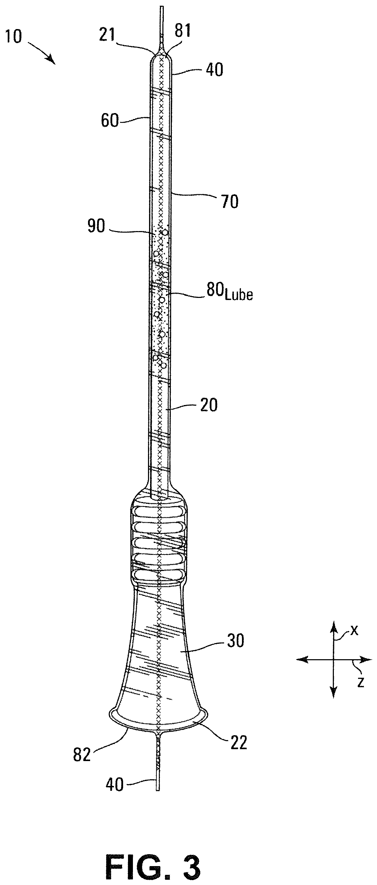

The invention is an efficiently packaged, ready to use intermittent urinary catheter **10.** Referring to Figures 1-3, the packaged catheter **10** includes, and in a preferred embodiment only includes the functional components of, (i) an intermittent urinary catheter **20** equipped with a fixture **30** at one end, (ii) packaging **70** formed from a base layer **40** and cover layer **60** of film, and (iii) a lubricant **90.**

The catheter **20** has an axial length **20x** with an insertion end **21,** an insertion end axial length portion **21x,** a fixture end **22,** and a fixture end axial length portion **22x.** The catheter **20** defines a top view profile **20_{Pf}**. The packaging **70** may be used with substantially any commercially available catheter **20,** but is particularly suited for use with shorter female urinary catheters.

The catheter **20** is hermetically packaged within a retention chamber **80** formed between base layer **40** and cover layer **60** films sealed together, preferably by heat seal, within a peripheral margin **75** of the packaging **70.** The films **40** and **60** may have originated from separate and independent rolls or sheets of film, or they may have been formed by simply folding a single length of film back upon itself. The packaging **70** formed by the base layer **40** and cover layer **60** films defines an outer peripheral edge **70_{Py}**. The retention chamber **80** has a periphery **80_{Py}** defining a top view profile **80_{Pf}** that generally conforms to the top view profile **20_{Pf}** of the catheter **20,** except for an optional enlarged compartment **80_{Lube}** intermediate the insertion **21** and funnel **22** ends of the catheter **20** in which lubricant **90** can be stored for coating the catheter **20** when it is withdrawn from the packaging **70** for use. The optional enlarged compartment **80_{Lube}** is preferably axially aligned with the balance of the retention chamber **80** such that the catheter **20** passes through the enlarged compartment **80_{Lube}.**

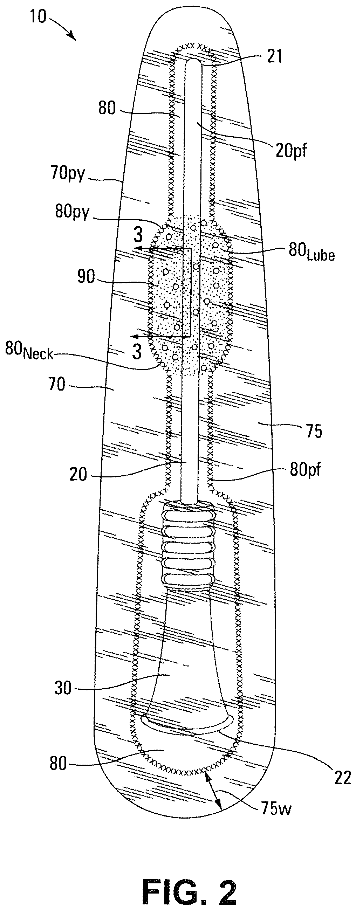

Referring to Figures 1 and 2, the margins **75** have a preferred width **75w** of between 0.2 and 5 cm, most preferred between about 0.3 and 2 cm, around the entire periphery of the retention chamber **80.**

The packaging layers **40** and **60** may be constructed from the same or different films, with the films selected from materials that are impervious to the lubricant **90,** and suitable for hermetically sealing the catheter **20** within a retention chamber **80** formed from the films **40** and **60.** Suitable materials include specifically but not exclusively, films of polyester, polyethylene, polypropylene, Surlyn^{®}, Tyvek^{®}, aluminum, Mylar^{®}, etc.

Referring to Figures 1 and 2, the retention chamber **80** has a top view profile **80_{Pf}** that tightly matches the top view profile **20_{Pf}** of the catheter **20** along a predominant portion of the axial length **20x** of the catheter **20** for purposes of maintaining lubricant **90** retained within the retention chamber **80** in close proximity to the catheter **20** so that the lubricant **90** will adhere to and coat the insertion end portion **21x** of the catheter **20** as the catheter **20** is withdrawn from the packaging **70.** The profiles are preferably matched such that the axial cross-sectional area of the retention chamber **80** (*i.e.,* the cross-sectional area of the retention chamber **80** in a plane extending in the lateral **y** and transverse **z** directions and perpendicular to the longitudinal **x** axis of the catheter **20)** is between 1.2 and 1.8 times the corresponding axial cross-sectional area of the catheter **20** along at least 50%, preferably along at least 80% and preferably along at least 90% of the axial length of the insertion end portion **21x** of the catheter **20.**

Referring to Figure 2, when the retention chamber **80** includes an expanded lubricant containing compartment **80_{Lube}**, the axial cross-sectional area of the lubricant containing compartment **80_{Lube}** is between 2 and 4 times the largest axial cross-sectional area of the catheter **20** lying within the compartment **80_{Lube}** as packaged. The lubricant containing compartment **80_{Lube}** necks down at the longitudinal **x** end of the compartment **80_{Lube}** proximate the fixture end **22** of the catheter **20,** preferably necking down at both longitudinal x ends, to an axial cross-sectional area that is between 1.2 and 1.8 times the largest axial cross-sectional area of the catheter **20** lying within the compartment **80_{Lube}** as packaged. This necked down area **80_{Neck}** formed from the packaging films **40** and **60,** serves to meter the coating of lubricant **90** on the catheter **20** as the catheter **20** is withdrawn from the packaging **70** for use.

### METHOD OF PACKAGING READY TO USE CATHETER

A preferred method of packaging the ready to use intermittent urinary catheter **10** employs a novel lubricant placement and spreading technique.

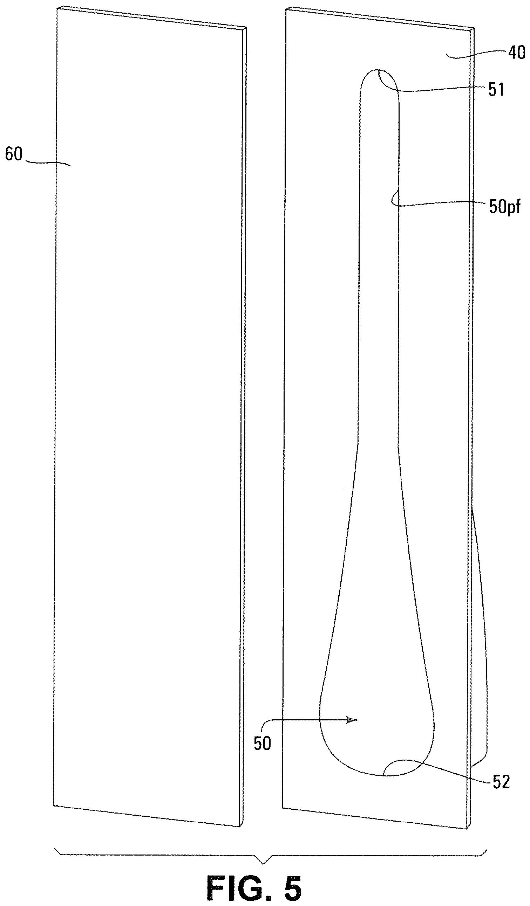

Referring to Figure 5, the method employs a base film **40,** a cover film **60** and lubricant **90.**

The base film **40** has a blister or pocket **50** with a first end **51** and a second end **52.** The pocket **50** preferably has a top view profile **50_{Pf}** conforming to the top view profile **20_{Pf}** of the intermittent urinary catheter **20,** and is configured to retain at least a portion and preferably the entire urinary catheter **20** within the pocket **50,** with the insertion end **21** of the catheter **20** proximate the first end **51** of the pocket **50** and the fixture end **22** of the catheter **20** proximate the second end **52** of the pocket.

The cover film **60** is preferably a planar sheet.

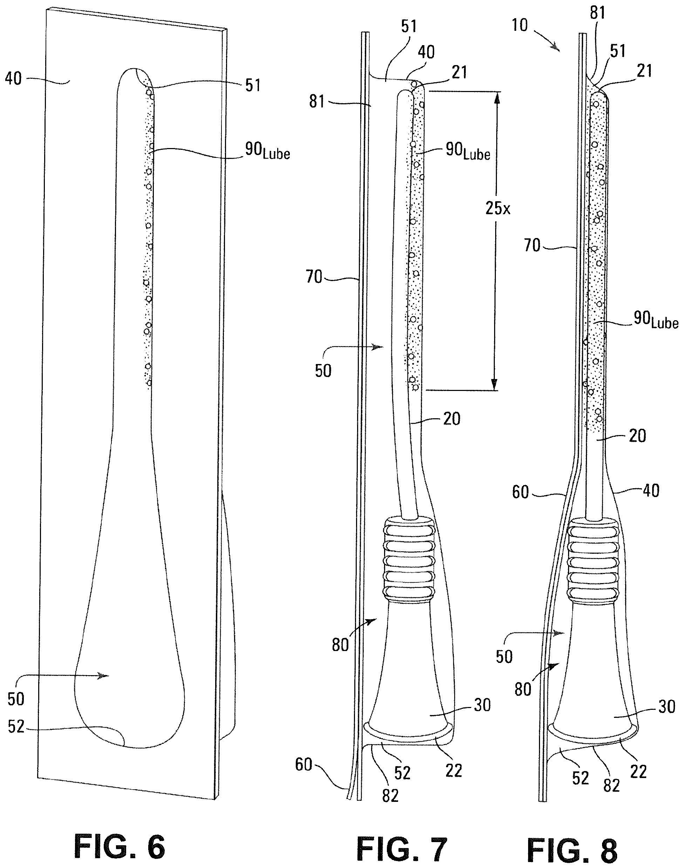

Referring to Figure 6, an amount of lubricant **90** is inserted into the pocket **50** in the base film **40** along a substantial axial length **x** of the insertion end portion **21x** of the pocket **50** so as to form a lubricant containing pocket **50.** The lubricant **90** is preferably applied so as to extend along at least about 30% of the axial length of the insertion end portion **21x** of catheter **20,** with a preference for a continuously applied axial x length of at least 50% of the axial length of the insertion end portion **21x,** more preferably at least 70% and most preferably at least 80%.

Referring to Figure 7, a urinary catheter **20** is then placed into the lubricant containing pocket **50** with an alpha axial length **25x** of the insertion end portion **21x** resting atop and in fluid communication with the lubricant **90** so as to form a catheter containing pocket **50,** with the insertion end **21** of the urinary catheter **20** proximate the first end **51** of the pocket **50** and the fixture end **22** of the urinary catheter **20** proximate the second end **52** of the pocket **50.** The alpha axial length **25x** of the insertion end portion **21x** resting atop and in fluid communication with the lubricant **90** is preferably between 30% and 90% of the insertion end length of the catheter, more preferably between 50% and 80% and most preferably 40% and 60%.

The cover film **60** is placed over the base film **40** and sealed, such as by heat sealing, to the base film **40** around the profile of the pocket **50_{Pf}** except along a length proximate the second end **52** of the pocket **50** so as to form a catheter containing retention chamber **80** between the base film **40** and the cover film **60** which is sealed except for an opening proximate the second end **82.**

Referring to Figure 8, a vacuum is drawn on the catheter containing retention chamber **80** through the opening sufficient to compress the retention chamber **80** and thereby cause lubricant **90** to circumferentially flow around and coat a substantial percentage of the surface area of the alpha axial length **25x** of the insertion end portion **21x** resting atop and in fluid communication with the lubricant **90,** with a preference for coating at least 70% of the surface area of the alpha axial length **25x** and most preferably 90%.

Pulling of a vacuum with resultant compression of the retention chamber **80** will also tend to draw lubricant **90** an axial distance **x** towards the second end **52** of the pocket **50.** The vacuum should be applied at a pressure and for a duration sufficient to effect substantially complete circumferential coating of that portion of the insertion end portion **21x** of the catheter **20** placed atop the lubricant **90,** but without causing the lubricant **90** to travel an axial distance **x** that results in coating any portion of the fixture end **22** of the catheter **20.**

The lubricant **90** preferably has a viscosity of between about 15,000 and about 500,000 mPa s at 22°C (between about 15,000 and about 500,000 centipoise at 72°F). When the viscosity is less than about 15,000 mPa s (15,000 cp) it is difficult to control the axial **x** length of the insertion end portion **21x** of catheter **20** which is coated with lubricant **90** when pulling the vacuum, along with an increased likelihood that lubricant **90** will migrate into contact with the fixture end portion **22x** of the catheter **20** under normal storage and transport conditions. Generally, when a high lubricity embodiment is desired the lubricant **90** preferably has a viscosity of between 15,000 and 50,000 mPa s at 22°C (15,000 and 50,000 centipoise at 72°F), and when superior control over lubricant **90** coverage during packaging and lubricant **90** migration during normal storage and handling is desired the lubricant **90** preferably has a viscosity of between 100,000 and 400,000 mPa s at 22°C (100,000 and 400,000 centipoise at 72°F).

The opening into the retention chamber **80** is then sealed, such as by heat sealing, to form a hermetically packaged intermittent urinary catheter **10** having a lubricated insertion end portion **21x** and a lubricant-free fixture end portion **22x.** The vacuum may be and preferably is at least partially released and more preferably is fully released prior to sealing of the opening.

## Claims

1. A method of hermetically packaging an intermittent urinary catheter (20) having an insertion end (21), an insertion end length defining an exterior surface area, a fixture end (22), and a fixture end length, comprising the steps of:
(a) obtaining a base film (40) having a pocket (50) with a first end (51) and a second end (52), wherein the pocket (50) is configured to retain the intermittent urinary catheter (20) with the insertion end (21) proximate the first end (51) and the fixture end (22) proximate the second end (52),
(b) injecting a limited amount of lubricant (90) into the pocket (50) proximate the first end (51) of the pocket so as to form a lubricant containing pocket (50),
(c) placing the intermittent urinary catheter (20) into the lubricant containing pocket (50) so as to form a catheter containing pocket (50), with the insertion end (21) of the intermittent urinary catheter (20) proximate the first end (51) and the fixture end (22) of the intermittent urinary catheter (20) proximate the second end (52),
(d) sealing a cover film (60) to the base film (40) with an unsealed opening proximate the second end (52) of the pocket (50) so as to form an enclosed catheter containing retention chamber (80) accessible through the opening,
(e) drawing a vacuum on the enclosed catheter containing retention chamber (80) through the opening sufficient to compress the retention chamber and cause lubricant (90) to circumferentially flow around and coat a portion of the exterior surface area of the insertion end length of the catheter (20) within the retention chamber (80) without reaching and coating the fixture end length of the catheter (20) within the retention chamber (80), and thereafter
(f) sealing the opening so as to form a hermetically packaged intermittent urinary catheter having a circumferentially lubricated insertion end length and a lubricant-free fixture end length.

2. The method of claim 1 wherein the intermittent urinary catheter (20) has a profile and the pocket (50) has a profile conforming to the profile of the intermittent urinary catheter (20).

3. The method of claim 1 wherein an alpha length (25x) of the intermittent urinary catheter (20) is placed atop and into fluid contact with the lubricant (90) within the pocket (50) in step (c) and the vacuum is sufficient to effect a circumferential flow of lubricant (90) around the alpha length (25x) so as to coat at least 70% of the external surface area of the alpha length (25x) of the catheter (20).

4. The method of claim 3 wherein the vacuum is sufficient to effect a circumferential flow of lubricant (90) around the alpha length (25x) so as to coat at least 90% of the external surface area of the alpha length (25x) of the catheter (20).

5. The method of claim 3 wherein the alpha length (25x) is between 30% and 90% of the insertion end length of the catheter (20).

6. The method of claim 3 wherein the alpha length (25x) is between 50% and 80% of the insertion end length of the catheter (20).

7. The method of claim 3 wherein the alpha length (25x) is between 40% and 60% of the insertion end length of the catheter (20).

8. The method of claim 1 wherein the lubricant (90) has a viscosity of between 15,000 and 500,000 mPa s at 22°C (15,000 and 500,000 centipoise at 72°F).

9. The method of claim 1 wherein the lubricant (90) has a viscosity of between 15,000 and 50,000 mPa s at 22°C (15,000 and 50,000 centipoise at 72°F).

10. The method of claim 1 wherein the lubricant (90) has a viscosity of between 100,000 and 400,000 mPa s at 22°C (100,000 and 400,000 centipoise at 72°F).

11. The method of claim 1 wherein the cover film (60) is heat sealed to the base film (40) in step (d).

12. The method of claim 1 wherein the opening is heat sealed.

13. The method of claim 1 wherein the cover film (60) is a planar sheet.

## Patentansprüche

1. Verfahren zum hermetischen Verpacken eines intermittierenden Harnkatheters (20), der ein Einführungsende (21), eine Einführungsendlänge, die einen Außenflächenbereich definiert, ein Befestigungsende (22) und eine Befestigungsendlänge aufweist, das die Schritte aufweist:
(a) Erhalten eines Basisfilms (40), der eine Tasche (50) mit einem ersten Ende (51) und einem zweiten Ende (52) aufweist, wobei die Tasche (50) konfiguriert ist, den intermittierenden Harnkatheter (20) mit dem Einführungsende (21) nahe dem ersten Ende (51) und dem Befestigungsende (22) nahe dem zweiten Ende (52) zu halten,
(b) Einspritzen einer begrenzten Menge Schmiermittel (90) in die Tasche (50) nahe dem ersten Ende (51) der Tasche, um eine Schmiermittel enthaltende Tasche (50) zu bilden,
(c) Anordnen des intermittierenden Harnkatheters (20) in der Schmiermittel enthaltenden Tasche (50), um eine Katheter enthaltende Tasche (50) mit dem Einführungsende (21) des intermittierenden Harnkatheters (20) nahe dem ersten Ende (51) und dem Befestigungsende (22) des intermittierenden Harnkatheters (20) nahe dem zweiten Ende (52) zu bilden,
(d) Siegeln eines Abdeckfilms (60) auf den Basisfilm (40) mit einer unversiegelten Öffnung nahe dem zweiten Ende (52) der Tasche (50), um eine abgeschlossene, Katheter enthaltende Aufbewahrungskammer (80) zu bilden, die durch die Öffnung zugänglich ist,
(e) Ziehen eines Vakuums an der abgeschlossenen, Katheter enthaltenden Aufbewahrungskammer (80) durch die Öffnung, das ausreicht, die Aufbewahrungskammer zu komprimieren und zu bewirken, dass das Schmiermittel (90) in Umfangsrichtung um einen Abschnitt des Außenflächenbereichs der Einführungsendlänge des Katheters (20) in der Aufbewahrungskammer (80) fließt und diese beschichtet, ohne die Befestigungsendlänge des Katheters (20) in der Aufbewahrungskammer (80) zu erreichen und zu beschichten, und danach
(f) Versiegeln der Öffnung, um einen hermetisch verpackten intermittierenden Harnkatheter zu bilden, der eine in Umfangsrichtung geschmierte Einführungsendlänge und eine schmiermittelfreie Befestigungsendlänge aufweist.

2. Verfahren nach Anspruch 1, wobei der intermittierende Harnkatheter (20) ein Profil aufweist und die Tasche (50) ein Profil aufweist, das dem Profil des intermittierenden Harnkatheters (20) entspricht.

3. Verfahren nach Anspruch 1, wobei im Schritt (c) eine Alpha-Länge (25x) des intermittierenden Harnkatheters (20) auf und in Fluidkontakt mit dem Schmiermittel (90) in der Tasche (50) angeordnet wird, und das Vakuum ausreicht, einen Umfangsfluss des Schmiermittels (90) um die Alpha-Länge (25x) zu bewirken, um mindestens 70% des Außenflächenbereichs der Alpha-Länge (25x) des Katheters (20) zu beschichten.

4. Verfahren nach Anspruch 3, wobei das Vakuum ausreicht, einen Umfangsfluss des Schmiermittels (90) um die Alpha-Länge (25x) zu bewirken, um mindestens 90% des Außenflächenbereichs der Alpha-Länge (25x) des Katheters (20) zu beschichten.

5. Verfahren nach Anspruch 3, wobei die Alpha-Länge (25x) zwischen 30% und 90% der Einführungsendlänge des Katheters (20) beträgt.

6. Verfahren nach Anspruch 3, wobei die Alpha-Länge (25x) zwischen 50% und 80% der Einführungsendlänge des Katheters (20) beträgt.

7. Verfahren nach Anspruch 3, wobei die Alpha-Länge (25x) zwischen 40% und 60% der Einführungsendlänge des Katheters (20) beträgt.

8. Verfahren nach Anspruch 1, wobei das Schmiermittel (90) eine Viskosität zwischen 15000 und 500000 mPa s bei 22°C (15000 und 500000 Centipoise bei 72°F) aufweist.

9. Verfahren nach Anspruch 1, wobei das Schmiermittel (90) eine Viskosität zwischen 15000 und 50000 mPa s bei 22°C (15000 und 50000 Centipoise bei 72°F) aufweist.

10. Verfahren nach Anspruch 1, wobei das Schmiermittel (90) eine Viskosität zwischen 100000 und 400000 mPa s bei 22°C (100000 und 400000 Centipoise bei 72°F) aufweist.

11. Verfahren nach Anspruch 1, wobei der Abdeckfilm (60) im Schritt (d) auf den Basisfilm (40) heißgesiegelt wird.

12. Verfahren nach Anspruch 1, wobei die Öffnung heißgesiegelt wird.

13. Verfahren nach Anspruch 1, wobei der Abdeckfilm (60) eine ebene Bahn ist.

## Revendications

1. Procédé d'emballage hermétique d'un cathéter urinaire intermittent (20) ayant une extrémité d'insertion (21), une longueur d'extrémité d'insertion définissant une surface extérieure, une extrémité d'élément fixé à demeure (22) et une longueur d'extrémité d'élément fixé à demeure, comprenant les étapes de:
(a) obtention d'un film de base (40) ayant une poche (50) avec une première extrémité (51) et une seconde extrémité (52), où la poche (50) est configurée pour maintenir le cathéter urinaire intermittent (20) avec l'extrémité d'insertion (21) à proximité de la première extrémité (51) et l'extrémité d'élément fixé à demeure (22) à proximité de la seconde extrémité (52),
(b) injection d'une quantité limitée de lubrifiant (90) dans la poche (50) à proximité de la première extrémité (51) de la poche de manière à former une poche contenant du lubrifiant (50),
(c) mise en place du cathéter urinaire intermittent (20) dans la poche contenant du lubrifiant (50) de manière à former une poche contenant le cathéter (50), avec l'extrémité d'insertion (21) du cathéter urinaire intermittent (20) à proximité de la première extrémité (51) et l'extrémité d'élément fixé à demeure (22) du cathéter urinaire intermittent (20) à proximité de la seconde extrémité (52),
(d) scellement d'un film de couverture (60) au film de base (40) avec une ouverture non scellée à proximité de la seconde extrémité (52) de la poche (50) de manière à former une chambre de maintien contenant le cathéter enfermé (80) accessible par l'ouverture,
(e) application d'un vide sur la chambre de maintien contenant le cathéter enfermé (80) par l'ouverture suffisamment pour comprimer la chambre de maintien et amener le lubrifiant (90) à s'écouler suivant la circonférence autour de et recouvrir une partie de la surface extérieure de la longueur d'extrémité d'insertion du cathéter (20) à l'intérieur de la chambre de maintien (80) sans atteindre et recouvrir la longueur d'extrémité d'élément fixé à demeure du cathéter (20) à l'intérieur de la chambre de maintien (80), et ensuite
(f) scellement de l'ouverture de manière à former un cathéter urinaire intermittent emballé hermétiquement ayant une longueur d'extrémité d'insertion lubrifiée suivant la circonférence et une longueur d'extrémité d'élément fixé à demeure sans lubrifiant.

2. Procédé selon la revendication 1 dans lequel le cathéter urinaire intermittent (20) a un profil et la poche (50) a un profil se conformant au profil du cathéter urinaire intermittent (20).

3. Procédé selon la revendication 1 dans lequel une longueur alpha (25x) du cathéter urinaire intermittent (20) est placée au-dessus de et en contact fluide avec le lubrifiant (90) à l'intérieur de la poche (50) à l'étape (c) et le vide est suffisant pour effectuer un écoulement suivant la circonférence de lubrifiant (90) autour de la longueur alpha (25x) de manière à recouvrir au moins 70 % de la surface externe de la longueur alpha (25x) du cathéter (20).

4. Procédé selon la revendication 3 dans lequel le vide est suffisant pour effectuer un écoulement suivant la circonférence de lubrifiant (90) autour de la longueur alpha (25x) de manière à recouvrir au moins 90 % de la surface externe de la longueur alpha (25x) du cathéter (20).

5. Procédé selon la revendication 3 dans lequel la longueur alpha (25x) est entre 30 % et 90 % de la longueur d'extrémité d'insertion du cathéter (20).

6. Procédé selon la revendication 3 dans lequel la longueur alpha (25x) est entre 50 % et 80 % de la longueur d'extrémité d'insertion du cathéter (20).

7. Procédé selon la revendication 3 dans lequel la longueur alpha (25x) est entre 40 % et 60 % de la longueur d'extrémité d'insertion du cathéter (20).

8. Procédé selon la revendication 1 dans lequel le lubrifiant (90) a une viscosité entre 15 000 et 500 000 mPa.s à 22°C (15 000 et 500 000 centipoises à 72°F).

9. Procédé selon la revendication 1 dans lequel le lubrifiant (90) a une viscosité entre 15 000 et 50 000 mPa.s à 22°C (15 000 et 50 000 centipoises à 72°F).

10. Procédé selon la revendication 1 dans lequel le lubrifiant (90) a une viscosité entre 100 000 et 400 000 mPa.s à 22°C (100 000 et 400 000 centipoises à 72°F).

11. Procédé selon la revendication 1 dans lequel le film de couverture (60) est thermoscellé au film de base (40) à l'étape (d).

12. Procédé selon la revendication 1 dans lequel l'ouverture est thermoscellée.

13. Procédé selon la revendication 1 dans lequel le film de couverture (60) est une feuille plane.
